# EUROPÄISCHE PATENTSCHRIFT

(11) **EP 2 621 425 B1**
(45) Veröffentlichungstag und Bekanntmachung des Hinweises auf die Patenterteilung: **01.04.2020**
(21) Anmeldenummer: 11805752.0
(22) Anmeldetag: 24.09.2011
(51) Int. Cl.: A61F 9/007

(54) **STEUERUNGSVORRICHTUNG FÜR EIN OPHTHALMOCHIRURGISCHES SYSTEM**
CONTROL DEVICE FOR AN OPHTHALMOSURGICAL SYSTEM
DISPOSITIF DE COMMANDE POUR UN SYSTÈME DE CHIRURGIE OPHTALMOLOGIQUE

(30) Priorität: 30.09.2010 DE 102010047009
(43) Veröffentlichungstag der Anmeldung: 07.08.2013
(73) Patentinhaber: Carl Zeiss Meditec AG, 07745 Jena (DE)
(72) Erfinder: HAUGER, Christoph, 73431 Aalen (DE); KRAUS, Martin, 73460 Hüttlingen (DE)
(74) Vertreter: Carl Zeiss AG - Patentabteilung
(86) Internationale Anmeldenummer: PCT/DE2011/001777
(87) Internationale Veröffentlichungsnummer: WO 2012/041290

(56) Entgegenhaltungen:
- US-A1- 2008 319 451
- US-A1- 2009 306 581
- US-B1- 6 544 254

## Beschreibung

Die Erfindung betrifft eine Steuerungsvorrichtung für ein ophthalmochirurgisches System zur Phakoemulsifikation einer Augenlinse und ein ophthalmochirurgisches System mit einer solchen Steuerungsvorrichtung.

Zur Behandlung einer Augenlinsentrübung, welche in der Medizin als Grauer Star bezeichnet wird, gibt es mehrere chirurgische Techniken. Die am weitesten verbreitete Technik ist die Phakoemulsifikation, bei der eine dünne Nadel in die erkrankte Linse eingeführt und mit Ultraschall zu Schwingungen angeregt wird. Die vibrierende Nadel emulsifiziert in ihrer nächsten Umgebung die Linse derart, dass die entstehenden Linsenpartikel durch eine Leitung mittels einer Pumpe abgesaugt werden können. Dabei wird ein Spülfluid (Irrigationsfluid) zugeführt, wobei das Absaugen der Partikel und des Fluides durch eine Aspirationsleitung erfolgt, welche üblicherweise innerhalb der Nadel angeordnet ist. Ist die Linse vollständig emulsifiziert und entfernt worden, kann in den leeren Kapselsack eine neue künstliche Linse eingesetzt werden, so dass ein derart behandelter Patient wieder ein gutes Sehvermögen erreichen kann.

Pro Jahr werden in Deutschland etwa 600.000 Operationen dieser Art durchgeführt. Die Komplikationsrate ist relativ gering, jedoch erfordert sie viel Erfahrung beim durchführenden Chirurgen. Ferner ist der Zeitaufwand für das Emulsifizieren einer Augenlinse immer noch relativ hoch. Um die Operationszeit verkürzen zu können, ist es möglich, die schwingende Nadel mit einer größeren Amplitude arbeiten zu lassen. Wenn dies ohne Unterbrechung geschieht, ist dies gleichbedeutend mit einem höheren Energieeintrag. Die Folge davon ist aber eine stärkere Erwärmung in der Umgebung der schwingenden Nadel. Da die Nadel durch die Hornhaut gestochen wird, bedeutet ein höherer Energieeintrag eine stärkere Wärmebelastung der Hornhaut. Dies kann dazu führen, dass mindestens teilweise umliegendes Gewebe wie zum Beispiel die Hornhaut überhitzt und beschädigt wird. Arbeitet der Chirurg hingegen mit einer Nadel, welche nur mit einer kleinen Amplitude betrieben wird, so dass nur ein geringer Energieeintrag in die Linse und das angrenzende Gewebe erfolgt, kann das Risiko einer Schädigung des Gewebes verringert werden. Die Operationsdauer verlängert sich dann jedoch entsprechend. Falls eine relativ harte Augenlinse vorliegt, kann der Chirurg eine Emulsifizierung nur durch höheren Energieeintrag erreichen. Um ein Verbrennen des Gewebes wie der Hornhaut zu verhindern, müssen jedoch häufige Unterbrechungen bei der Operation eingelegt werden, so dass genügend Zeit für eine Abkühlung verfügbar ist.

Aus US 2009/0306581 A1 ist ein ophthalmochirurgisches System bekannt, bei dem mittels eines Operationsmikroskops Bilddaten einer chirurgischen Prozedur aufgenommen werden können. Aus US 6,544,254 B1 ist ein Verfahren zum Entfernen einer Augenlinse mittels einer Kombination aus Laserstrahlung und Ultraschallenergie bekannt.

Es besteht eine Aufgabe darin, eine Steuerungsvorrichtung sowie ein ophthalmochirurgisches System zu schaffen, bei der/dem eine Operation durch Phakoemulsifikation einer Augenlinse in kurzer Zeit möglich ist, ohne eine Verbrennung umliegenden Gewebes zu riskieren. Allgemein soll durch die Steuerungsvorrichtung und das ophthalmochirurgische System das Risiko für unerwünschte Schäden an einem Patientenauge während einer Kataraktoperation verringert werden.

Diese Aufgabe wird für die Steuerungsvorrichtung durch den Gegenstand des unabhängigen Patentanspruchs 1 gelöst. Vorteilhafte Weiterbildungen der Erfindung sind Gegenstand der Unteransprüche. Die Aufgabe für das ophthalmochirurgische System wird durch den Gegenstand des unabhängigen Anspruchs 11 gelöst.

Gemäß der Erfindung ist eine Steuerungsvorrichtung für ein ophthalmochirurgisches System zur Phakoemulsifikation einer Augenlinse vorgesehen, welche aufweist:
- ein optisches System, mit dem von einem Objektbereich ein Bild erzeugbar ist, wobei sich in dem Objektbereich mindestens ein Teil der zu emulsifizierenden Augenlinse und ein Teil einer Nadel eines Phakohandstückes anordnen lässt,
- eine Bildauswerteeinheit, welche geeignet ist, das erzeugte Bild so auszuwerten, dass mindestens eine Auswertegröße ermittelt wird, welche von einer Eigenschaft eines durch Phakoemulsifikation erzeugten Partikels der Augenlinse oder einer Relation des Partikels zu seiner Umgebung abhängig ist,
eine Steuereinheit, mit der in Abhängigkeit von der mindestens einen zugeführten Auswertegröße eine Steuergröße ermittelbar ist, wobei sich mittels der Steuergröße ein Betrag einer dem Phakohandstück mittels einer Energiequelle zugeführten Ultraschallenergie zur Phakoemulsifikation der Augenlinse steuern lässt.

Eine Ultraschallenergie, welche durch eine Nadel zur Augenlinse zugeführt wird, hängt somit von einer Auswertegröße ab, welche durch ein optisches System ermittelt werden kann. Die Auswertegröße ist abhängig von einer Eigenschaft eines Partikels der Augenlinse oder einer Relation des Partikels zu seiner Umgebung. Der Energieeintrag zur Zertrümmerung der Augenlinse ist somit abhängig von einer Eigenschaft eines Linsenpartikels, welches durch das optische System erfasst werden kann. Mit einer solchen Steuerungsvorrichtung kann die Erfahrung eines Chirurgen zwar nicht vollständig ersetzt werden, jedoch kann eine höhere Sicherheit bei der Operation erreicht werden. Bei einer bestimmten Eigenschaft eines Partikels oder einer Relation des Partikels zu seiner Umgebung kann ein Energieeintrag somit gering gewählt werden, so dass die Gefahr für eine Hornhautverbrennung ebenfalls gering ist. Falls durch die Auswertung des erzeugten Bildes eine Eigenschaft eines Partikels erkannt wird, welche einen hohen Energieeintrag erfordert, liegt dieser hohe Energieeintrag nur so lange an, wie diese Eigenschaft des Partikels gegeben ist.

Durch das optische System ist eine schnelle Bildverarbeitung möglich, so dass kurze Reaktionszeiten für die Steuerungsvorrichtung erreichbar sind. Damit kann erheblich schneller erkannt werden, welche Höhe des Energieeintrages sinnvoll ist, als wenn ein Chirurg die Steuerung des Energieeintrages manuell zum Beispiel durch ein Fußpedal betätigt, wie das gemäß dem Stand der Technik der Fall ist.

Es wird darauf hingewiesen, dass eine Verstopfung (Okklusion) einer Aspirationsleitung durch ein Partikel nicht erforderlich ist, um die Steuerungsvorrichtung betreiben zu können.

Vorzugsweise ist die Auswertegröße der Bildauswerteeinheit eine geometrische Messgröße eines Partikels der zu emulsifizierenden Augenlinse. Das optische System kann dabei so angeordnet sein, das eine Draufsicht auf die zu emulsifizierende Linse möglich ist. Wenn als geometrische Messgröße eines Partikels die Fläche des zu emulsifizierenden Partikels gewählt ist, kann somit die zuzuführende Energie zur Emulsifikation des Partikels von der Fläche dieses Partikels abhängig gemacht werden. Ein Partikel, welches in der Draufsicht eine Fläche in Anspruch nimmt, welche kleiner als der kleinste Innendurchmesser der Aspirationsleitung in der Nadel ist, durch welche die Partikel abgesaugt werden, ist bereits so klein, dass die Nadel nur mit einer geringen Energie oder gar keiner Energie betrieben zu werden braucht. Dies erhöht die Sicherheit gegen eine Überhitzung von Gewebe im Bereich der Nadel. Wenn die Fläche des Partikels jedoch größer als der kleinste Innendurchmesser der Aspirationsleitung ist, durch welche die Partikel abgesaugt werden, kann die Energiequelle so angesteuert werden, dass ein relativ hoher Betrag einer Ultraschallenergie zum Antrieb der Nadel zugeführt wird.

Eine andere geometrische Messgröße kann auch der Umfang des zu emulsifizierenden Partikels oder das Volumen des zu emulsifizierenden Partikels sein, wobei die letztere Messgröße ein dreidimensionales Erfassen der Linsengeometrie erfordert. Eine andere geometrische Messgröße kann auch ein Abstand eines Randes eines Partikels der zu emulsifizierenden Augenlinse zu einem vorderen Rand der Nadel bzw. einer Ansaugöffnung der Aspirationsleitung sein. Wenn das Partikel noch relativ weit von der Nadel entfernt ist, kann der Energieeintrag gering gewählt werden. Wenn ein vorbestimmter Mindestabstand zwischen dem Rand des zu emulsifizierenden Partikels und dem Rand der Nadel unterschritten ist, kann jedoch der Betrag der zugeführten Energie erhöht werden.

Gemäß einer anderen Ausführungsform der Erfindung kann die Auswertegröße auch eine optische Messgröße eines Partikels der zu emulsifizierenden Augenlinse sein. Aus Erfahrung weiß ein Arzt, dass ein sehr hell leuchtendes Partikel meistens weicher und somit einfacher zu emulsifizieren ist, als ein dunkles und härteres Partikel. Eine optische Auswertung des Bildes alternativ zur geometrischen Messgröße oder zusätzlich zur geometrischen Messgröße kann dabei helfen, den erforderlichen Energieeintrag festzulegen. Vorzugsweise ist die optische Messgröße eine Lichtdurchlässigkeit, eine Grauwertintensität oder eine Farbintensität.

Die Auswertegröße kann auch eine mechanische oder kinematische Messgröße eines Partikels der zu emulsifizierende Augenlinse sein. Die mechanische Messgröße kann die Härte des Partikels sein. Je härter ein Partikel ist, umso höher muss ein Energieeintrag sein, um das Partikel in kleine Teile zu emulsifizieren. Dies ist von Bedeutung, da bekannt ist, dass eine Augenlinse in der Regel keine konstante Härte innerhalb ihres gesamten Volumens besitzt, sondern es Zonen unterschiedlicher Härte in der Augenlinse gibt.

Das optische System weist vorzugsweise ein Lichtmikroskop und / oder ein OCT-System auf. Das OCT-System kann auch innerhalb eines Lichtmikroskopes angeordnet sein, so dass der Arzt durch ein Lichtmikroskop das Operationsgebiet sehen kann, während mittels des OCT-Systems der erforderliche Energieertrag steuerbar ist.

Gemäß einer Weiterbildung der Erfindung kann das optische System auch mindestens teilweise im Phakohandstück angeordnet sein. Vorzugsweise ist das optische System in diesem Fall ein OCT-System, wobei in das Phakohandstück eine Faser integriert ist. Die Faser kann vorzugsweise rotierend oder scannend ausgelegt sein, um einen Volumenbereich des am Rand der Nadel befindlichen Partikels zu erfassen.

Weitere Vorteile und Merkmale der Erfindung werden mit Bezug auf die nachfolgenden Zeichnungen erklärt, in welchen zeigen:
- Figur 1: eine schematische Darstellung einer ersten Ausführungsform eines ophthalmochirurgischen Systems mit einer Steuerungsvorrichtung gemäß der Erfindung;
- Figur 2: ein Bild einer Bildauswerteeinheit mit einem Teil einer Nadel und einem zu emulsifizierenden Partikel, wobei das Partikel von der Nadel weit entfernt ist;
- Figur 3: ein Bild einer Bildauswerteeinheit mit einem Teil einer Nadel und einem zu emulsifizierenden Partikel, wobei das Partikel sehr nahe an der Nadel angeordnet ist;
- Figur 4: ein Bild einer Bildauswerteeinheit mit einer Nadel und einem Partikel, wobei das Partikel die Nadel berührt;
- Figur 5: ein Bild einer Bildauswerteeinheit mit einem Teil einer Nadel und einem kleinen Partikel, wobei das Partikel weit von der Nadel entfernt ist;
- Figur 6: ein Bild einer Auswerteeinheit mit einem Teil einer Nadel und einem kleinen Partikel, welches die Nadel berührt;
- Figur 7: eine Tabelle zur Ermittlung einer Ultraschallenergie in Abhängigkeit von zwei geometrischen Messgrößen;
- Figur 8: eine Tabelle zur Ermittlung einer Ultraschallenergie in Abhängigkeit von zwei geometrischen Messgrößen und einer optischen Messgröße;
- Figur 9: ein Diagramm, welches schematisch eine Abhängigkeit zwischen einer optischen Messgröße und einer zugeführten Ultraschallenergie zeigt; und
- Figur 10: eine schematische Darstellung einer zweiten Ausführungsform eines ophthalmochirurgischen Systems mit einer Steuerungsvorrichtung gemäß der Erfindung.

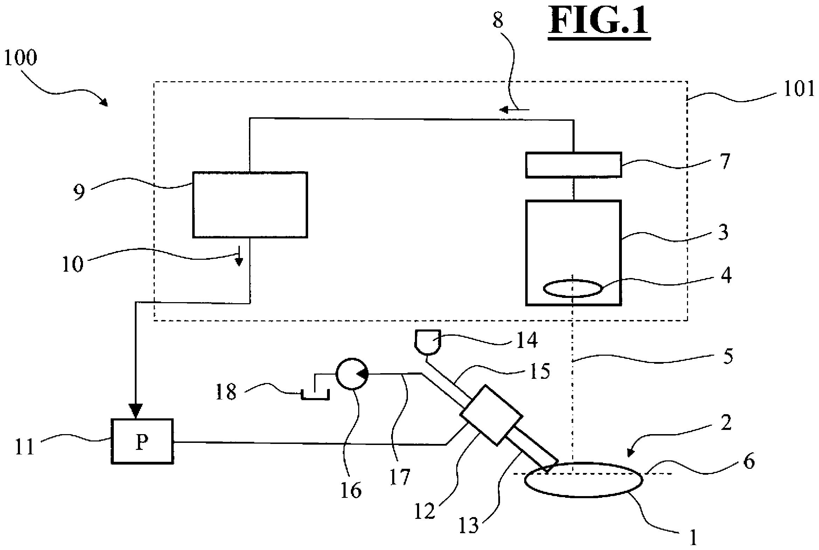

In Figur 1 ist ein ophthalmochirurgisches System 100 mit einer erfindungsgemäßen Steuerungsvorrichtung 101 dargestellt. Eine Augenlinse 1, welche zu emulsifizieren ist, liegt innerhalb eines Objektbereiches 2 eines optischen Systems 3. Das optische System 3 ist bei dieser Ausführungsform ein Lichtmikroskop, welches ein Hauptobjektiv 4 aufweist, welches eine optische Achse 5 besitzt. Eine Fokusebene 6 ist innerhalb des Objektbereiches 2 der Augenlinse 1 angeordnet. Das von dem optischen System 3 erzeugte Bild wird einer Bildauswerteeinheit 7 zugeführt, welche geeignet ist, das zugeführte Bild auszuwerten. Durch die Auswertung wird mindestens eine Auswertegröße 8 ermittelt, welche von einer Eigenschaft eines durch Emulsifikation zu erzeugenden Linsenpartikels oder einer Relation des Linsenpartikels zu seiner Umgebung abhängig ist. Die Auswertegröße 8 wird einer Steuereinheit 9 zugeführt, welche in Abhängigkeit von der mindestens einen zugeführten Auswertegröße eine Steuergröße 10 ermittelt. Die Steuergröße kann einer Energiequelle 11 und von dort einem Phakohandstück 12 zugeführt werden, so dass ein Betrag einer von der Energiequelle 11 dem Phakohandstück 12 zugeführten Ultraschallenergie gesteuert werden kann. Die Steuergröße 10 kann auch direkt dem Handstück 12 zugeführt und dort mit der von der Energiequelle 11 gelieferten Energie verarbeitet werden.

Die dem Phakohandstück 12 bzw. den zugehörigen Aktuatoren zugeführte Ultraschallenergie wird an eine Nadel 13 übertragen, mit welcher die Augenlinse 1 emulsifiziert werden kann. Bei der Emulsifikation der Augenlinse 1 wird von einem Irrigationsbehälter 14 ein Irrigationsfluid durch eine Irrigationsleitung 15 zum Phakohandstück bis an den vorderen Bereich der Nadel geleitet. Mittels einer Aspirationspumpe 16 werden die emulsifizierten Partikel und Fluid durch eine Aspirationsleitung 17 zu einem Aspirationsbehälter 18 abgesaugt.

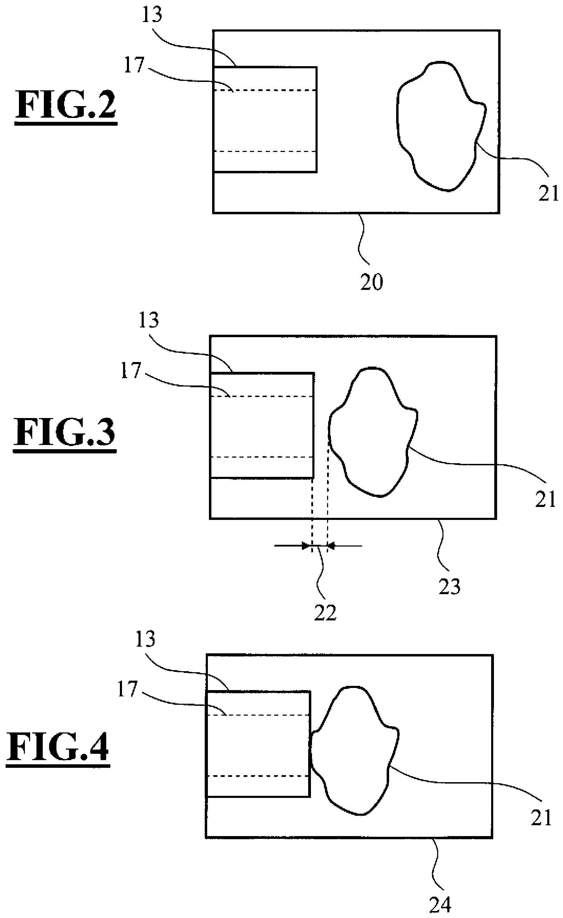

Figur 2 zeigt ein Bild 20 einer Bildauswerteeinheit 7, in welchem ein Teil einer Nadel 13 mit einer darin enthaltenen Aspirationsleitung 17 und ein vor der Nadel 13 zu emulsifizierendes Partikel 21 dargestellt ist. Das Partikel 21 ist noch relativ weit von der Nadel entfernt. In Figur 3 ist ein Bild 23 dargestellt, wobei das Partikel 21 jedoch in einem relativ nahen Abstand 22 zur Nadelspitze 13 angeordnet ist. Die Bildauswerteeinheit kann dieses Bild 23 nun derart auswerten, das der Abstand 22 erfasst wird und die einer Energiequelle zuzuführende Ultraschallenergie entsprechend gesteuert wird. Wenn der Abstand 22 zum Beispiel kleiner als 100 Mikrometer ist, kann die Nadel 13 derart angesteuert werden, dass sie einen größeren Energiebetrag zugeführt bekommt. Somit wird nur der höhere Energiebetrag zugelassen, wenn eine Okklusion unmittelbar bevorsteht oder eingetreten ist.

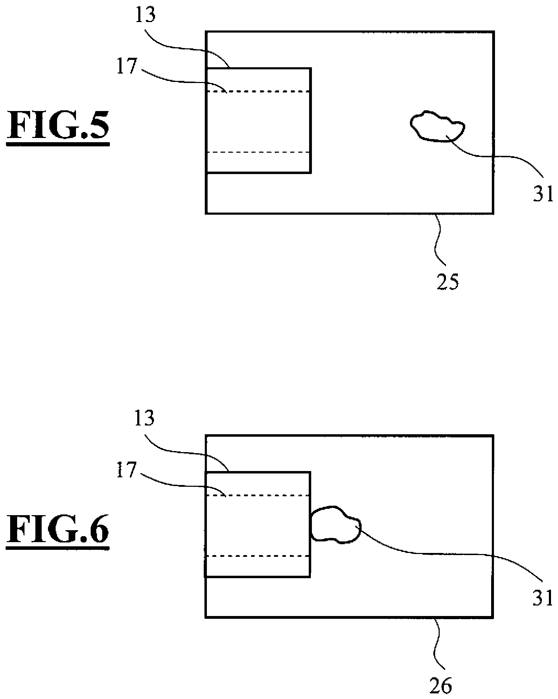

In Figur 4 ist ein Bild 24 einer Bildauswerteeinheit 7 dargestellt, wobei ein vorderer Teil der Nadel 13 mit darin enthaltener Aspirationsleitung 17 und ein zu emulsifizierendes Partikel 21 dargestellt sind, wobei das Partikel 21 direkt in Kontakt mit der Nadel 13 steht. Auch in diesem Fall ist der Abstand 22 kleiner als zum Beispiel 100 Mikrometer, so dass die Nadel mit einer relativ hohen Ultraschallenergie betrieben werden kann, um das Partikel 21 zu zerkleinern. Wenn dies gelungen ist und aus dem Partikel 21 mehrere kleinere Partikel 31 gebildet worden sind, siehe Figur 5, kann die Ultraschallenergie wieder auf einen niedrigeren Wert gesteuert werden.

In Figur 5 ist ein Bild 25 mit einem Partikel 31 dargestellt, welches relativ weit von der Nadel 13 entfernt ist. In diesem Fall ist zudem die von dem Partikel 31 gebildete Fläche in der Draufsicht so klein, das zu erwarten ist, dass das Partikel problemlos durch die Absaugöffnung der Aspirationsleitung 13 abgesaugt werden kann. Bei einem solchen Bild kann die Steuerungsvorrichtung so betrieben werden, dass die zugeführte Ultraschallenergie einen relativ niedrigen Betrag annimmt.

Bei der in Figur 6 dargestellten Situation ist ein Bild 26 zu sehen, bei dem das Partikel 31 direkt die Nadel 13 berührt. Das Partikel 31 ist jedoch so klein, dass es problemlos durch die Aspirationsleitung 17 der Nadel 13 abgesaugt werden kann, so dass auch der zugeführte Betrag einer Ultraschallenergie relativ niedrig gehalten werden kann.

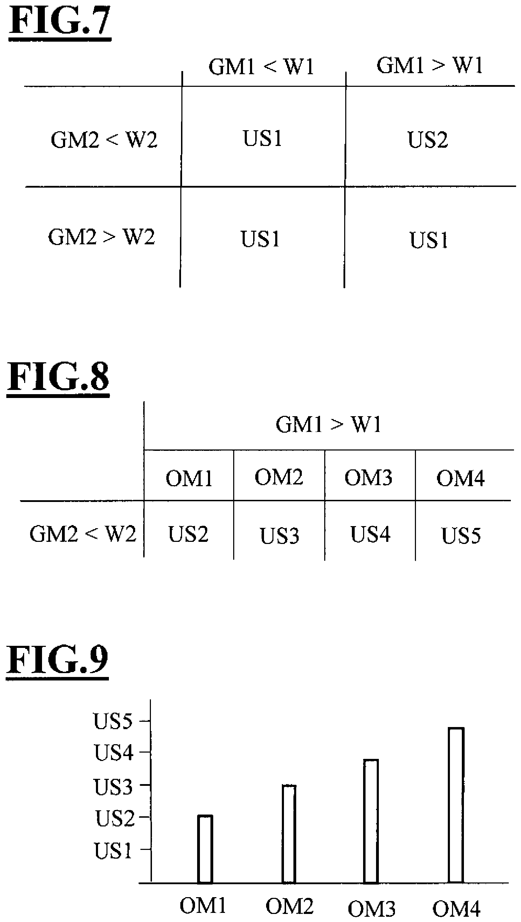

In Figur 7 ist eine Tabelle dargestellt, in welcher die Wechselwirkung zwischen einem zuzuführenden Betrag einer Ultraschallenergie und einer ersten geometrischen Messgröße GM1 sowie einer zweiten geometrischen Messgröße GM2 aufgeführt ist. Die erste geometrische Messgröße GM1 kann zum Beispiel eine Fläche eines zu emulsifizierenden Partikels innerhalb eines Bildes sein, welches von dem optischen System erzeugt wird. Die zweite geometrische Messgröße GM2 kann ein Abstand eines zu emulsifizierenden Partikels von dem vorderen Rand der Aspirationsleitung 17 sein. Wenn GM1 kleiner als eine vorbestimmte Größe W1 ist, wobei W1 zum Beispiel die Querschnittsfläche im Bereich des kleinsten Innendurchmessers einer Aspirationsleitung 17 ist, ergeben sich in Abhängigkeit von der zweiten geometrischen Messgröße GM2 bestimmte Vorgaben für eine zuzuführende Ultraschallenergie an die Nadel 13. Die zweite geometrische Messgröße GM2 kann von einem zweiten Schwellwert W2 abhängig gemacht werden. Wenn GM2 kleiner W2 ist, bedeutet dies, dass der Abstand zwischen dem zu emulsifizierenden Partikel und der Nadel 13 relativ klein ist. Wenn GM2 größer W2 ist, bedeutet dies, dass das Partikel relativ weit von der Nadel 13 entfernt ist. W2 kann zum Beispiel die maximale Amplitude der Nadel 13 sein.

Gemäß Figur 7 ergeben sich somit folgende Situationen:
Wenn GM1 kleiner W1 und GM2 kleiner W2 ist, wenn also ein relativ kleines Partikel in einem sehr kleinen Abstand zur Nadel im Bild einer Bildauswerteeinheit 7 erfasst wird, siehe zum Beispiel Fig. 6, kann ein erster Betrag US1 einer Ultraschallenergie zugeführt werden. Dieser Betrag kann einen Wert von null oder nahezu null besitzen. Wenn hingegen GM1 größer W1 ist, wobei GM2 unverändert kleiner W2 ist, liegt somit ein relativ großes zu emulsifizierendes Partikel relativ nahe an einer Nadel 13 an, vgl. Fig. 4. In diesem Fall kann die Steuerungsvorrichtung so betrieben werden, dass ein höherer Betrag einer Ultraschallenergie US2 an eine Nadel zugeführt wird, so dass das relativ große Partikel in möglichst kurzer Zeit in kleine Partikel zertrümmert wird.

Wenn GM1 kleiner W1 und GM2 größer W2 ist, wenn also ein relativ kleines Partikel in großer Entfernung zur Nadel 13 angeordnet ist, vgl. Fig. 5, kann ebenfalls nur mit einem niedrigen Betrag US1 an Ultraschallenergie gearbeitet werden. Falls gemäß einer anderen Situation GM1 größer W1 und GM2 größer W2 ist, falls also ein relativ großes Partikel noch relativ weit von der Nadel entfernt ist, vgl. Fig. 2, kann auch hier mit einem niedriegen Betrag US1 an Ultraschallenergie gearbeitet werden. Dies bedeutet, dass gemäß Figur 7 ein hoher Energieeintrag nur in dem Fall vorgesehen wird, wenn sich ein großes Partikel relativ nahe oder in Kontakt mit einer Nadel 13 befindet.

In Figur 8 ist eine Tabelle dargestellt, in welcher die jeweiligen Beträge an Ultraschallenergie in Abhängigkeit von einer ersten geometrischen Messgröße GM1, einer zweiten geometrischen Messgröße GM2 und unterschiedlichen Beträgen einer optischen Messgröße dargestellt sind. Bei der optischen Messgröße kann es sich zum Beispiel um die Lichtdurchlässigkeit eines Partikels handeln. Bei der in Figur 8 dargestellten Tabelle ist eine optische Messgröße in vier Stufen eingeteilt, wobei OM1 eine hohe Transparenz bedeutet, OM2 eine geringere Transparenz bedeutet, OM3 eine noch geringere Transparenz bedeutet und OM4 eine nahezu nicht mehr vorhandene Transparenz bedeutet. Wenn GM1 größer W1 ist, wenn also ein relativ großes Partikel vorliegt, und gleichzeitig GM2 kleiner W2 ist, wenn also das Partikel relativ nahe an einer Nadel ist, kann die zugeführte Ultraschallenergie von der vorhandenen Transparenz des Partikels abhängig gemacht werden. Wenn das Partikel eine hohe Transparenz besitzt, kann mit einem relativ niedrigen Betrag US2 an Ultraschallenergie gearbeitet werden. Wenn das Partikel eine niedrigere Transparenz besitzt, also die optische Messgröße einen Betrag OM2 besitzt, kann mit einem höheren Betrag US3 einer Ultraschallenergie gearbeitet werden. Falls die Transparenz noch geringer ist, so dass eine optische Messgröße mit dem Betrag OM3 vorliegt, kann mit einem noch höheren Ultraschallenergiebetrag US4 gearbeitet werden. Falls bei der zu emulsifizierenden Linse ein Partikel erkannt wird, welches nahezu keine Transparenz mehr aufweist, so dass eine optische Messgröße vom Betrag OM4 ermittelt wird, kann für den Fall, dass dieses Partikel relativ nahe an der Nadel angeordnet ist, also GM2 kleiner W2 ist, ein sehr hoher Ultraschallenergiebetrag US5 zugeführt werden. Die Begründung für diese Stufung liegt darin, dass davon ausgegangen wird, dass mit abnehmender Transparenz das zu emulsifizierende Partikel härter ist und mehr Energie aufgebracht werden muss, um es in kleinere Partikel zu zertrümmern.

In Figur 9 sind diese in Figur 8 dargestellten Situationen noch einmal anschaulicher in Form eines Balkendiagrammes dargestellt. Aus Figur 9 ist ersichtlich, dass mit geringer werdender Transparenz ein zunehmend höherer Ultraschallenergiebetrag zugeführt werden kann.

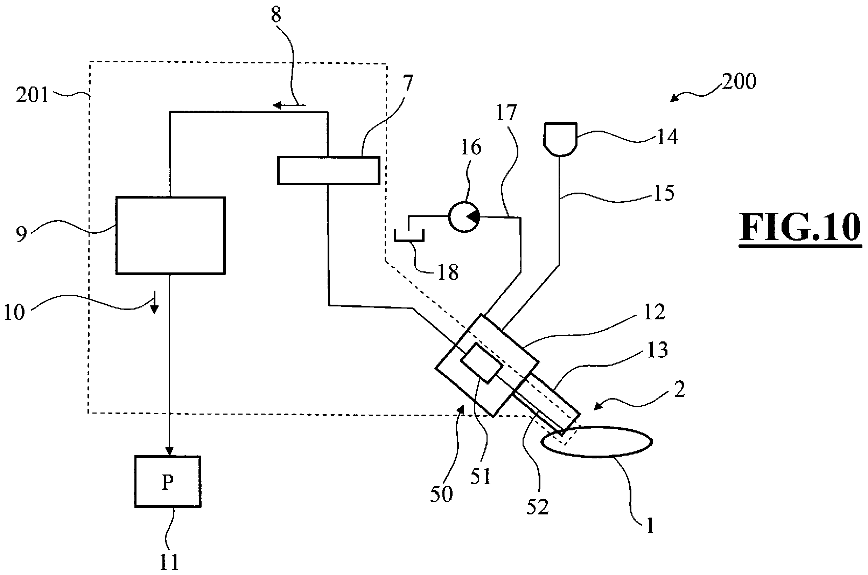

In Figur 10 ist eine zweite Ausführungsform einer Steuerungsvorrichtung 201 für ein ophthalmochirurgischen System 200 dargestellt. Das ophthalmochirurgischen System 200 weist eine Steuerungsvorrichtung 201 auf, welche ein optisches System 50, eine Bildauswerteeinheit 7 und eine Steuereinheit 9 aufweist. Das optische System 50 kann in Form eines OCT-Systems vorliegen, welches eine Quelle für ein zeitlich inkohärentes und räumlich kohärentes Licht aufweist, zusätzlich eine Vorrichtung 51 zur Überlagerung von Laserstrahlung aus einem Probenstrahlengang mit Laserstrahlung aus einem Referenzstrahlengang aufweist, und ferner eine Bilderzeugungseinheit aufweist, um ein Bild zu erzeugen. Zu dem optischen System 50 gehört ferner noch eine OCT-Faser 52, welche in der Nadel 13 oder an der Nadel 13 angeordnet ist. Das von der Bilderzeugungseinheit erzeugte Bild wird daraufhin zu einer Bildauswerteeinheit 7 geleitet, welche das Bild auswertet, wobei eine Auswertegröße 8 ermittelt wird. Diese Auswertegröße 8 kann einer Steuereinheit 9 zugeführt werden, welche eine Steuergröße 10 ermittelt. Die Steuergröße 10 kann anschließend auf eine Energiequelle 11 einwirken, so dass dem Phakohandstück 12 bzw. der Nadel 13 des Phakohandstückes 12 eine zugehörige Ultraschallenergie zugeführt werden kann. Das ophthalmochirurgische System 200 weist ferner einen Irrigationsbehälter 14 auf, aus welchem durch eine Irrigationsleitung 15 Fluid zum Handstück 12 und dort zu einer zu behandelnden Augenlinse 1 geleitet werden kann. Die emulsifizierten Partikel können dann mittels einer Pumpe 16 durch eine Aspirationsleitung 17 zu einem Aspirationsbehälter 18 abgesaugt werden.

Es ist ferner denkbar, dass ein ophthalmochirurgischen System ein optisches System 3 in Form eines Lichtmikroskops und ein optisches System 50 in Form eines OCT-Systems aufweist.

Gemäß einer weiteren Ausführungsform kann auch innerhalb eines Lichtmikroskopes ein OCT-System angeordnet sein, wobei zusätzlich innerhalb eines Phakohandstückes ein derartiges OCT-System angeordnet sein kann.

## Patentansprüche

1. Steuerungsvorrichtung (101; 201) für ein ophthalmochirurgisches System (100; 200) zur Phakoemulsifikation einer Augenlinse (1), aufweisend:
- ein optisches System (3; 50), mit dem von einem Objektbereich (2) ein Bild (20, 23, 24, 25, 26) erzeugbar ist, wobei sich in dem Objektbereich (2) mindestens ein Teil der zu emulsifizierenden Augenlinse (1) und ein Teil einer Nadel (13) eines Phakohandstückes (12) anordnen lässt,
- eine Bildauswerteeinheit (7), welche geeignet ist, das erzeugte Bild (20, 23, 24, 25, 26) so auszuwerten, dass mindestens eine Auswertegröße (8) ermittelt wird, welche von einer Eigenschaft eines durch Phakoemulsifikation erzeugten Partikels (21, 31) der Augenlinse (1) oder einer Relation des Partikels (21, 31) zu seiner Umgebung abhängig ist,
- eine Steuereinheit (9), mit der in Abhängigkeit von der mindestens einen zugeführten Auswertegröße (8) eine Steuergröße (10) ermittelbar ist,
wobei sich mittels der Steuergröße (10) ein Betrag einer dem Phakohandstück (12) mittels einer Energiequelle (11) zugeführten Ultraschallenergie zur Phakoemulsifikation der Augenlinse (1) steuern lässt.

2. Steuerungsvorrichtung (101; 201) nach Anspruch 1, wobei die Auswertegröße (8) der Bildauswerteeinheit (7) eine geometrische Messgröße eines Partikels (21, 31) der zu emulsifizierenden Augenlinse (1) ist.

3. Steuerungsvorrichtung (101; 201) nach Anspruch 2, wobei die geometrische Messgröße die Fläche oder der Umfang oder das Volumen des zu emulsifizierenden Partikels in dem erzeugten Bild (20, 23, 24, 25, 26) ist.

4. Steuerungsvorrichtung (101; 201) nach einem der Ansprüche 1 bis 3, wobei die Auswertegröße (8) ein Abstand (22) eines Randes eines Partikels (21, 31) der zu emulsifizierenden Augenlinse (1) zu einem vorderen Rand der Nadel (13) ist.

5. Steuerungsvorrichtung (101; 201) nach einem der vorhergehenden Ansprüche, wobei die Auswertegröße (8) eine optische Messgröße eines Partikels (21, 31) der zu emulsifizierenden Augenlinse (1) ist.

6. Steuerungsvorrichtung (101; 201) nach Anspruch 5, wobei die optische Messgröße eine Lichtdurchlässigkeit, eine Grauwertintensität oder eine Farbintensität ist.

7. Steuerungsvorrichtung (101; 201) nach einem der vorhergehenden Ansprüche, wobei die Auswertegröße (8) eine mechanische oder kinematische Messgröße eines Partikels (21, 31) der zu emulsifizierenden Augenlinse (1) ist.

8. Steuerungsvorrichtung (101; 201) nach Anspruch 7, wobei die mechanische Messgröße die Härte des Partikels (21, 31) ist.

9. Steuerungsvorrichtung (101; 201) nach einem der vorherigen Ansprüche, wobei das optische System (3; 50) ein Lichtmikroskop und/oder ein OCT-System aufweist.

10. Steuerungsvorrichtung (201) nach einem der vorherigen Ansprüche, wobei das optische System (50) mindestens teilweise im Phakohandstück (12) angeordnet ist.

11. Ophthalmochirurgisches System (100; 200), welches eine Steuerungsvorrichtung (101; 201) nach einem der Ansprüche 1 bis 10 aufweist.

## Claims

1. Control device (101; 201) for an ophthalmic surgical system (100; 200) for phacoemulsification of an eye lens (1), having:
- an optical system (3; 50), by means of which an image (20, 23, 24, 25, 26) can be generated of an object region (2), wherein at least part of the eye lens (1) to be emulsified and part of a needle (13) of a phacoemulsification handpiece (12) can be arranged in the object region (2),
- an image evaluation unit (7), which is suitable for evaluating the generated image (20, 23, 24, 25, 26) in such a way that at least one evaluation variable (8) is established, which is dependent on a property of a particle (21, 31), produced by phacoemulsification, of the eye lens (1) or on a relation of the particle (21, 31) to its surroundings,
- a control unit (9), by means of which, dependent on the at least one supplied evaluation variable (8), it is possible to establish a control variable (10), wherein the control variable (10) can control an absolute value of ultrasound energy, supplied to the phacoemulsification handpiece (12) by means of an energy source (11), for phacoemulsification of the eye lens (1).

2. Control device (101; 201) according to Claim 1, wherein the evaluation variable (8) from the image evaluation unit (7) is a geometric measurement variable of a particle (21, 31) of the eye lens (1) to be emulsified.

3. Control device (101; 201) according to Claim 2, wherein the geometric measurement variable is the area or the circumference or the volume of the particle to be emulsified in the generated image (20, 23, 24, 25, 26).

4. Control device (101; 201) according to one of Claims 1 to 3, wherein the evaluation variable (8) is a distance (22) of an edge of a particle (21, 31) of the eye lens (1) to be emulsified from a front edge of the needle (13).

5. Control device (101; 201) according to one of the preceding claims, wherein the evaluation variable (8) is an optical measurement variable of a particle (21, 31) of the eye lens (1) to be emulsified.

6. Control device (101; 201) according to Claim 5, wherein the optical measurement variable is a light transmissivity, a grayscale-value intensity or a color intensity.

7. Control device (101; 201) according to one of the preceding claims, wherein the evaluation variable (8) is a mechanical or kinematic measurement variable of a particle (21, 31) of the eye lens (1) to be emulsified.

8. Control device (101; 201) according to Claim 7, wherein the mechanical measurement variable is the hardness of the particle (21, 31).

9. Control device (101; 201) according to one of the preceding claims, wherein the optical system (3; 50) has a light microscope and/or an OCT system.

10. Control device (201) according to one of the preceding claims, wherein the optical system (50) is at least partly arranged within the phacoemulsification handpiece (12).

11. Ophthalmic surgical system (100; 200), which has a control device (101; 201) according to one of Claims 1 to 10.

## Revendications

1. Dispositif de commande (101 ; 201) pour un système ophtalmo-chirurgical (100 ; 200) destiné à la phaco-émulsification d'un cristallin (1), ledit dispositif comprenant :
- un système optique (3 ; 50) permettant de générer une image (20, 23, 24, 25, 26) à partir d'une zone objet (2), au moins une partie du cristallin (1) à émulsifier et une partie d'une aiguille (13) d'une pièce à main phaco (12) pouvant être disposées dans la zone objet (2),
- une unité d'évaluation d'image (7) qui est appropriée pour évaluer l'image générée (20, 23, 24, 25, 26) de façon à déterminer au moins une grandeur d'évaluation (8) qui dépend d'une propriété d'une particule (21, 31), générée par phaco-émulsification, du cristallin (1) ou d'une relation de la particule (21, 31) avec son environnement,
- une unité de commande (9) permettant de déterminer une grandeur de commande (10) en fonction de l'au moins une grandeur d'évaluation fournie (8),
la grandeur de commande (10) pouvant être utilisée pour commander une quantité d'énergie ultrasonore, fournie à la pièce à main phaco (12) au moyen d'une source d'énergie (11), pour la phaco-émulsification du cristallin (1).

2. Dispositif de commande (101 ; 201) selon la revendication 1, la grandeur d'évaluation (8) de l'unité d'évaluation d'image (7) étant une grandeur de mesure géométrique d'une particule (21, 31) du cristallin (1) à émulsifier.

3. Dispositif de commande (101 ; 201) selon la revendication 2, la grandeur de mesure géométrique étant la surface ou la circonférence ou le volume de la particule à émulsifier dans l'image générée (20, 23, 24, 25, 26).

4. Dispositif de commande (101 ; 201) selon l'une des revendications 1 à 3, la grandeur d'évaluation (8) étant une distance (22) d'un bord d'une particule (21, 31) du cristallin (1) à émulsifier à un bord avant de l'aiguille (13).

5. Dispositif de commande (101 ; 201) selon l'une des revendications précédentes, la grandeur d'évaluation (8) étant une grandeur de mesure optique d'une particule (21, 31) du cristallin (1) à émulsifier.

6. Dispositif de commande (101 ; 201) selon la revendication 5, la grandeur de mesure optique étant une transmittance lumineuse, une intensité de valeur de gris ou une intensité de couleur.

7. Dispositif de commande (101 ; 201) selon l'une des revendications précédentes, la grandeur d'évaluation (8) étant une grandeur de mesure mécanique ou cinématique d'une particule (21, 31) du cristallin (1) à émulsifier.

8. Dispositif de commande (101 ; 201) selon la revendication 7, la grandeur de mesure mécanique étant la dureté de la particule (21, 31).

9. Dispositif de commande (101 ; 201) selon l'une des revendications précédentes, le système optique (3 ; 50) comportant un microscope optique et/ou un système OCT.

10. Dispositif de commande (201) selon l'une des revendications précédentes, le système optique (50) étant disposé au moins partiellement dans la pièce à main phaco (12).

11. Système ophtalmo-chirurgical (100 ; 200), lequel comporte un dispositif de commande (101 ; 201) selon l'une des revendications 1 à 10.
